# EUROPÄISCHE PATENTSCHRIFT

(11) **EP 2 316 460 B1**
(45) Veröffentlichungstag und Bekanntmachung des Hinweises auf die Patenterteilung: **23.04.2014**
(21) Anmeldenummer: 11001347.1
(22) Anmeldetag: 12.05.2005
(51) Int. Cl.: A61K 45/06, A61K 33/14, A61K 9/08, A61K 31/7004, A61M 1/28

(54) **Lösung für die Peritonealdialyse**
Solution for peritoneal dialysis
Solution pour la dialyse péritoneale

(30) Priorität: 13.05.2004 DE 102004023828
(43) Veröffentlichungstag der Anmeldung: 04.05.2011
(62) Teilanmeldung aus: 05745322.7
(73) Patentinhaber: Fresenius Medical Care Deutschland GmbH, 61352 Bad Homburg v.d.H. (DE)
(72) Erfinder: Passlick-Deetjen, Jutta, 35392 Giessen (DE); Schaub, Thomas P. Dr. med., 61352 Bad Homburg (DE); Topp, Georg, 35321 Laubach (DE)
(74) Vertreter: Herrmann, Uwe

(56) Entgegenhaltungen:
- EP-A- 0 602 585
- EP-A- 0 935 967
- EP-A- 1 038 552
- US-A1- 2002 077 580
- NAKAYAMA M ET AL: "Anti-hypertensive effect of low Na concentration (120 mEq/l) solution for CAPD patients", CLINICAL NEPHROLOGY 1994 GERMANY, Bd. 41, Nr. 6, 1994, Seiten 357-363, XP009057567, ISSN: 0301-0430

## Beschreibung

Die Erfindung betrifft eine Lösung für die Peritonealdialyse nach dem Oberbegriff des Anspruchs 1.

In einer Peritonealdialyselösung sind im allgemeinen neben einem Puffersystem und einem Osmotikum Elektrolyte enthalten, wobei es sich hier üblicherweise um Calcium-, Natrium- und Magnesiumsalze handelt, die häufig in Form von Chloriden eingesetzt werden.

Während der Dialysebehandlung kommt es zu einem Übertritt von zahlreichen Blutbestandteilen, unter anderem von Natriumionen aus dem Blut des Patienten, über das Peritoneum in das Dialysat. Die Menge des auf diese Weise aus dem Blut entfernten Natrium hängt unter anderem von dem Konzentrationsgradienten von Natrium zwischen der Blutseite und der Dialysatseite ab.

Allgemein bekannt ist die Tatsache, dass Bluthochdruckpatienten vom Genuss von Kochsalz (NaCl) abgeraten wird. Eine Blutdruckabsenkung ist einerseits durch eine kochsalzarme Ernährung und andererseits durch einen effektiven Natriumentzug während der Dialyse zu erreichen. Um den Natriumgehalt des Extrazellulärraumes während der Dialyse zu verringern, um eine Blutdruckabsenkung zu erzielen, ist vorgeschlagen worden, eine Peritonealdialyselösung mit geringem Natriumgehalt einzusetzen (Clin. Neph. Vol. 41(6) (1994) pp. 357-363, Nakayama et al.). Die aus dieser Druckschrift bekannte Peritonealdialyselösung weist einen Natriumgehalt von 120 mmol/l auf. Weitere Komponenten sind Calciumionen, Magnesiumionen (diese Kationen werden üblicherweise mit Chlorid als Anion verabreicht), Lactat sowie Glucose. Der Einsatz dieser Peritonealdialyselösung bringt eine signifikante Reduktion des extrazellulären Natriumgehalts sowie des Blutdruckes mit sich, was darauf zurückgeführt werden kann, dass Natriumionen aufgrund des erhöhten Konzentrationsgradienten in erheblichem Umfang durch Diffusion und nicht nur durch konvektiven Transport abgezogen werden.

Der Einsatz der vorbekannten Peritonealdialyselösung hat jedoch unerwünschte Nebenwirkungen, die einerseits auf eine mangelnde Entwässerung der Patienten und andererseits auf eine Hyponaträmie zurückzuführen sind. Insbesondere bei Patienten mit geringer oraler Natriumaufnahme ist die alleinige Anwendung der genannten Peritonealdialyselösung mit einer Natriumkonzentration von 120 mmol/l mit erheblichen Nachteilen (Hyponaträmie) verbunden und daher inakzeptabel.

Aus den Patentschriften U.S. 5,589,197, U.S. 5,629,025 und U.S. 5,631,025 sind Peritonealdialyselösungen bekannt, die unterschiedliche Osmotika enthalten und deren Natriumgehalt im Bereich zwischen 35 und 125 mmol/l liegt. Als bevorzugter Konzentrationsbereich wird eine Natriumionen-Konzentration von ≤ 120 mmol/l angegeben. Mit einer derartigen Peritonealdialyselösung wurde klinisch ebenfalls eine Absenkung des Blutdruckes sowie eine Steigerung des Ultrafiltrationsvolumens bei gleicher Osmolarität gegenüber vorbekannten Standardlösungen mit üblichen Natriumkonzentrationen im Bereich von 134 mmol/l festgestellt. Allerdings ergeben sich auch bei der Verwendung der aus den US-Patentschriften bekannten Peritonealdialyselösungen die oben beschriebenen auf den zu geringen Natriumgehalt zurückzuführenden klinischen Nachteile.

Bei vorbekannten Peritonealdialyselösungen stellt sich somit entweder das Problem des Bluthochdruckes (bei einer Natriumionen-Konzentration von ca. 134 mmol/l) oder das Problem, dass mit klinischen Nebenwirkungen zu rechnen ist (bei einer Natriumionen-Konzentration ≤ 120 mmol/l). Bei der heute üblichen Peritonealdialyse wird in der Regel eine Natriumionen-Konzentration im Bereich von 134 mmol/l eingestellt, da sich auf diese Weise die beschriebenen Nebenwirkungen von Dialyselösungen mit geringem Natriumgehalt vermeiden lassen. Das sich gleichzeitig ergebende Problem des Bluthochdruckes wird medikamentös behandelt.

Es ist die Aufgabe der vorliegenden Erfindung, eine Lösung für die Peritonealdialyse bereitzustellen, die einen blutdrucksenkenden Effekt hat und bei der die durch den Einsatz von Peritonealdialyselösungen mit geringem Natriumgehalt bekannten Nebenwirkungen nicht auftreten.

Diese Aufgabe wird durch eine Lösung für die Peritonealdialyse mit den Merkmalen des Anspruchs 1 gelöst. Danach liegt die Natriumionen-Konzentration der Peritonealdialyselösung bei 125 mmol/l.

Natrium wird üblicherweise in Form von Kochsalz (NaCl), d.h. als Chlorid eingesetzt. Eine Reduktion der Natriumionenkonzentration kann daher zu einer äquimolaren Reduktion der Chloridionenkonzentration führen. Ein synergistischer Effekt kann aufgrund der im Falle der Verwendung von Natrium als Chlorid zwangsläufig ebenfalls abgesenkten Chloridionenkonzentration nicht ausgeschlossen werden. Die erfindungsgemäße Lösung für die Peritonealdialyse ermöglicht bei Vermeidung von Nebenwirkungen eine effektive Blutdruckabsenkung, wobei diese Effekte bei einem Natiumionen-Gehalt in dem beanspruchten Bereich und besonders ausgeprägt bei 125 mmol/l und 91-94 mmol/l Chloridionengehalt auftreten. Die erfindungsgemäße Peritonealdialyselösung führt zu einer Steigerung der Salzausscheidung und dient der Unterstützung der oralen Salz-Diätetik. Antihypertensive Mittel können aufgrund der blutdrucksenkenden Wirkung der erfindungsgemäßen Lösung bei mehr als 50% der Peritonealdialysepatienten eingespart werden.

In weiterer Ausgestaltung der Erfindung ist vorgesehen, dass die Lösung für die Peritonealdialyse ferner ein Osmotikum, weitere Elektrolyte sowie einen Puffer aufweist.

Aus der EP 0 935 967 A2 ist es bekannt, eine Peritonealdialyselösung bestehend aus zwei Einzellösungen bereit zu stellen, wobei die erste Einzellösung Calciumionen, weitere Elektrolytsalze und Glucose enthält und mit einer physiologisch verträglichen Säure auf einen pH-Wert unter 3,2 angesäuert ist und wobei die zweite Einzellösung Bicarbonat mit einem Gehalt von ≤ 10 mmol/l und das Salz einer schwachen Säure mit pka < 5 enthält. Eine derartige Peritonealdialyselösung bringt den Vorteil mit sich, dass einerseits der Abbau von Glucose während der Hitzesterilisation verhindert wird und dass andererseits aufgrund des geringen CO₂-Partialdruckes keine besonderen Anforderungen an das Beutelmaterial hinsichtlich der CO₂-Barriereeigenschaften zu stellen sind.

Eine derartige aus zwei Einzellösungen bestehende Lösung für die Peritonealdialyse kommt auch für die vorliegende Erfindung in Betracht.

Dementsprechend kann vorgesehen sein, dass die Lösung aus zwei Einzellösungen besteht und dass die erste Einzellösung das Osmotikum und eine physiologisch verträgliche Säure und die zweite Einzellösung einen Puffer aufweist.

Besteht die Lösung für die Peritonealdialyse aus zwei Einzellösungen, kann vorgesehen sein, dass in jeder der Einzellösungen Natriumionen vorliegen. Dabei ist das Mischungsverhältnis derart einzustellen, dass die Natriumionen-Konzentration der Peritonealdialyselösung, das heißt der fertigen Anwendungslösung bei 125 mmol/l liegt.

Werden zwei Einzellösungen verwendet, kann vorgesehen sein, dass in einer ersten Einzellösung außer Calciumionen weitere Elektrolyte, das Osmotikum (beispielsweise Glucose) sowie eine physiologisch verträgliche Säure vorliegen. In einer zweiten Einzellösung kann das Puffersystem vorgelegt werden. Dies besteht aus Bicarbonat. Entsprechend der Lehre der EP 0 935 967 A2 werden günstige Ergebnisse erzielt, wenn die erste Einzellösung auf einen pH-Wert unter 3,2 angesäuert ist und wenn die Bicarbonatkonzentration der zweiten Einzellösung 10 mmol/l nicht überschreitet. Auf den Inhalt der EP 0 935 967 A2 wird Bezug genommen.

Durch ein geeignetes Mischungsverhältnis der ersten und zweiten Einzellösung lässt sich ohne weiteres der erfindungsgemäße Konzentrationsbereich der Natriumionen der Anwendungslösung einstellen.

Gemäß der Erfindung ist eine rein Bicarbonat-gepufferte Lösung vorgesehen. Dementsprechend ist vorgesehen, dass der Puffer aus einem Hydrogencarbonat-Puffer besteht. Dabei kann vorgesehen sein, dass die Lösung aus zwei Einzellösungen besteht. Die erste Einzellösung kann Natriumionen, Calciumionen, Magnesiumionen, H⁺-Überschußionen, Chloridionen und Glucose und die zweite Einzellösung Natriumionen und Hydrogencarbonationen enthalten.

Die erste Einzellösung kann wie folgt zusammengesetzt sein:

| | |
|---|---|
| Natriumionen [mmol/l]: | 172-200 |
| Calciumionen [mmol/l]: | 2-4 |
| Magnesiumionen [mmol/l]: | 0,8-1,2 |
| H⁺-Überschuss [mmol/l]: | 0,9-1,1 |
| Chlorid [mmol/l]: | 176-210 |
| Glucose [mmol/l]: | 100-500 |

Die zweite Einzellösung kann den Bicarbonat-Puffer enthalten und folgende Zusammensetzung aufweisen:

| | |
|---|---|
| Natriumionen [mmol/l]: | 70-80 |
| Hydrogencarbonat [mmol/l]: | < 80 |

Werden zwei Einzellösungen verwendet, werden diese üblicherweise hitzesterilisiert, anschließend zu der Anwendungslösung zusammengeführt und sodann dem Patienten verabreicht. Statt zwei können auch mehr als zwei Einzellösungen Verwendung finden, sofern dies im Einzelfall sinnvoll ist.

Ebenso ist es denkbar, nur eine Einzellösung vorzusehen und in dieser Natriumionen in dem erfindungsgemäßen Konzentrationsbereich vorzulegen.

Die vorliegende Erfindung betrifft ferner eine Lösung nach Anspruch 3, wobei die Einzellösungen in einem Doppelkammerbeutel bereitgestellt sind, der aus einem Kunststoffbeutel besteht, in dem eine erste Kammer mit der ersten Einzellösung und eine zweite Kammer mit der zweiten Einzellösung benachbart zueinander angeordnet sind, wobei beide Kammern durch eine Schweißnaht voneinander abgetrennt sind, die derart dimensioniert ist, dass sie sich bei Druck auf eine der mit Flüssigkeit gefüllten Kammern öffnet, so dass der Inhalt der beiden Kammern miteinander vermischbar ist. In einem derartigen Doppelkammerbeutel können das Puffersystem beispielsweise bestehend aus Lactat und Bicarbonat in einer Beutelkammer gelagert werden, während Glucose und die Elektrolyte in einer zweiten Kammer in saurem Milieu gelagert werden können. Nach der Sterilisation werden die Beutelinhalte miteinander gemischt und dem Patienten zugeführt. Dabei sind die Natriumionen-Konzentrationen der Einzellösungen so zu wählen, dass sich in der fertigen Anwendungslösung die erfindungsgemäße Natriumionen-Konzentration ergibt.

Weitere Einzelheiten und Vorteile der Erfindung werden anhand des im folgenden dargestellten Ausführungsbeispiels näher erläutert:

| Phase II-Studie | CAPD Niedrig-Natrium Lösung FME, 2003 | | | CAPD Niedrig-Natrium Lösung Nakayama et al, 1994 | | |
|---|---|---|---|---|---|---|
| Zeit (Wochen) | W 0 | W +2 | W +4 | W 0 | W +2 | W +4 |
| Patienten | N=4 | N=4 | N=4 | N=9 | N=8 | N=7 |
| D-Natrium (mmol/l) | 134 | 125 | 125 | 132 | 120 | 120 |
| D-Chlorid (mmol/l) | 102.5 | 93.5 | 53.5 | 96 | 84 | 84 |
| MAD (mmHg) | 116 | 105 | 108 | 123 | 116 | 107 |
| KG (kg) | 65.5 | 65.6 | 64.8 | 61 | 61.7 | 61.6 |
| Mittleres S-Natrium (mmol/l) | 138.8 | 35.5 | 137.0 | 137 | 133 | 136 |
| Mittleres UF-Volumen (ml/Tag) | 1088 | n.b. | 1068 | 918 | 880 | 890 |
| Mittlere TPN-Ausscheidung (mmol/Tag) | 83.2 | n.b. | 55.1 | 38 | n.b. | 85 |
| SUE | Keine | | | 2x 'drop-out': | | |
| | | | | 1x Hyponatriämie, 1x Überwässerung | | |

| | | | | | | |
|---|---|---|---|---|---|---|
| **Legende**: FME, Fresenius Medical Care; W, Woche; N, Anzahl der Patienten; D-, Dialysat; S-, Serum; MAD, mittlerer arterieller (Blut-)Druck (Formel: MAD = (SBD-DBD) / 3+DBD; SBD, systolischer Blutdruck; DBD, diastolischer Blutdruck; KG, Körpergewicht; UFV, Ultrafiltrations-Volumen; TPNA, transperitoneale Natrium-Ausscheidung; SUE, schweres unerwünschtes Ereignis; n.b., nicht bestimmt. | | | | | | |

Die Tabelle zeigt das Ergebnis einer Studie, bei der Dialysepatienten mit der erfindungsgemäßen Peritonealdialyselösung behandelt wurden (Spalten 2-4). im Vergleich dazu sind die Ergebnisse der aus in Clin. Neph. Vol. 41(6) (1994) pp. 357-363, Nakayama et al. entnehmbaren Studie angegeben (Spalten 5-7).

In der zweiten Zeile ist die Behandlungsdauer in Wochen (W) sowie die Anzahl (N) der zu dem jeweiligen Zeitpunkt teilnehmenden Patienten angegeben.

Wie sich aus der zweiten und fünften Spalte ergibt, wurden die Patienten beim Start der Studie mit der erfindungsgemäßen Dialyselösung (W 0) auf 134 mmol/l Natriumgehalt, bei der Studie nach Nakayama et al. auf 132 mmol/l Natriumgehalt eingestellt. Die Chloridionenkonzentration der erfindungsgemäßen Peritonealdialyselösung betrug 93,5 mmol/l.

Sodann wurde die Natriumionen-Konzentration im Dialysat auf 125 mmol/l gemäß der vorliegenden Erfindung eingestellt und mit den veröffentlichten Werten von 120 mmol/l gemäß Nakayama et al. verglichen.

Aus Zeile 5 der Tabelle ist ersichtlich, dass bei Verwendung der erfindungsgemäßen Dialyselösung eine deutliche Reduktion des Blutdruckes (MAD) erzielt werden konnte. Nach zweiwöchiger Behandlungszeit (W+2) ergab sich trotz der höheren Natriumkonzentration der erfindungsgemäßen Dialyselösung als in der Vergleichslösung absolut und relativ eine stärke Blutdruckabsenkung als bei dem Vergleichsversuch gemäß Nakayama et al. Nach vierwöchiger Behandlungsdauer ergaben sich trotz der höheren Natrium-Konzentration der erfindungsgemäßen Lösung vergleichbare Blutdruckwerte wie bei dem Vergleichsversuch.

Das mittlere Ultrafiltrationsvolumen (UF-Volumen) lag bei der erfindungsgemäßen Lösung in einer ähnlichen Größenordnung wie bei der zu Versuchsbeginn angewandten Lösung (134 mmol/l Natrium-Gehalt).

Wie sich aus der letzten Zeile der Tabelle ergibt, ergab die Anwendung der erfindungsgemäßen Peritonealdialyselösung keinerlei Nebenwirkungen bzw. schwere unerwünschte Ereignisse. Im Gegensatz dazu mussten bei den Versuchen gemäß Nakayama et al. zwei Patienten die Studie abbrechen. In einem Fall wurde eine Überwässerung festgestellt, in einem anderen Fall wurde die Behandlung aufgrund einer Hyponaträmie und den sich daraus ergebenden Nebenwirkungen beendet. Die erfindungsgemäße Dialyselösung ermöglicht somit eine Peritonealdialyse, bei der sich die von vorbekannten Dialyselösungen mit geringem Natriumgehalt bekannten Nebenwirkungen vermeiden lassen und gleichzeitig eine effektive Blutdruckabsenkung erzielbar ist.

## Patentansprüche

1. Lösung für die Peritonealdialyse, erhältlich durch das Zusammenführen zweier Einzellösungen, wobei die erste Einzellösung ein Osmotikum und eine physiologisch verträgliche Säure, und die zweite Einzellösung einen Puffer aufweist,
**dadurch gekennzeichnet,**
**dass** die erste Einzellösung Natriumionen, Calciumionen, Magnesiumionen, H⁺-Überschussionen, Chloridionen und Glucose aufweist,
**dass** die zweite Einzellösung Natriumionen in einem Konzentrationsbereich von 70 bis 80 mmol/l und Hydrogencarbonationen in einem Konzentrationsbereich von weniger als 80 mmol/l aufweist, wobei das Puffersystem ausschließlich aus einem Hydrogencarbonat-Puffer besteht, und
**dass** die fertige Lösung Natriumionen in einer Konzentration von 125 mmol/l enthält und Chloridionen in einer Konzentration im Bereich von 91 mmol/l bis 94 mmol/l enthält.

2. Lösung gemäß Anspruch 1, **dadurch gekennzeichnet, dass** die erste Einzellösung folgende Bestandteile umfasst:
| | |
|---|---|
| Natriumionen: | 172 bis 200 mmol/l |
| Calciumionen: | 2 bis 4 mmol/l |
| Magnesiumionen: | 0,8 bis 1,2 mmol/l |
| H⁺-Überschuss: | 0,9 bis 1,1 mmol/l |
| Chlorid: | 176 bis 210 mmol/l |
| Glucose: | 100 bis 500 mmol/l. |

3. Lösung für die Peritonealdialyse gemäß einem der vorangehenden Ansprüche,
**dadurch gekennzeichnet,**
**dass** die Einzellösungen in einem Doppelkammerbeutel bereitgestellt sind, der aus einem Kunststoffbeutel besteht, der benachbart zueinander eine erste Kammer enthaltend die erste Einzellösung und eine zweite Kammer enthaltend die zweite Einzellösung aufweist, wobei die Kammern durch eine Schweißnaht voneinander abgetrennt sind, die derart dimensioniert ist, dass sie sich bei Druck auf einer der flüssigkeitsgefüllten Kammern öffnet und sich der Inhalt der beiden Kammern miteinander vermischt.

## Claims

1. A solution for peritoneal dialysis obtainable by combining two separate solutions, wherein the first separate solution contains an osmotic agent and a physiologically compatible acid and the second separate solution contains a buffer,
**characterized in that**
the first separate solution contains sodium ions, calcium ions, magnesium ions, H⁺ excess ions, chloride ions and glucose;
the second separate solution contains sodium ions in a concentration in the range from 70 to 80 mmol/l and hydrogen carbonate ions in a concentration in the range of less than 80 mmol/l, wherein the buffer system consists exclusively of a hydrogen carbonate buffer; and
the finished solution contains sodium ions in a concentration of 125 mmol/l and chloride ions in a concentration in the range from 91 mmol/I to 94 mmol/l.

2. A solution in accordance with claim 1, **characterized in that** the first separate solution comprises the following components:
| | |
|---|---|
| Calcium ions: | 2 to 4 mmol/l |
| Magnesium ions: | 0.8 to 1.2 mmol/l |
| H⁺ excess: | 0.9 to1.1 mmol/l |
| Chloride: | 176 to 210 mmol/l |
| Glucose: | 100 to 500 mmol/l |

3. A solution for peritoneal dialysis in accordance with one of the preceding claims,
**characterized in that**
the separate solutions are provided in a double-chambered pouch which consists of a plastic pouch having a first chamber containing the first separate solution and a second chamber containing the second separate solution arranged adjacent to one another, with the chambers being separated from one another by a weld seam dimensioned such that it opens on pressure onto one of the chambers filled with liquid and the contents of the two chambers are mixed with one another.

## Revendications

1. Solution pour la dialyse péritonéale, réalisable en réunissant deux solutions individuelles, la première solution individuelle comportant un osmoticum et un acide physiologiquement compatible, et la seconde solution individuelle comportant un tampon,
**caractérisée en ce que**
la première solution individuelle comporte des ions sodium, des ions calcium, des ions magnésium, des ions H⁺ en excès, des ions chlorure et du glucose,
**en ce que** la seconde solution individuelle comporte des ions sodium dans une plage de concentration de 70 à 80 mmol/l et des ions hydrogénocarbonate dans une plage de concentration de moins de 80 mmol/l, le système de tampon étant composé exclusivement d'un tampon hydrogénocarbonate, et
**en ce que** la solution finie contient des ions sodium en une concentration de 125 mmol/l et contient des ions chlorure en une concentration située dans la plage allant de 91 mmol/l à 94 mmol/l.

2. Solution selon la revendication 1, **caractérisée en ce que** la première solution individuelle comprend les composants suivants :
| | |
|---|---|
| ions sodium : | 172 à 200 mmol/l |
| ions calcium : | 2 à 4 mmol/l |
| ions magnésium : | 0,8 à 1,2 mmol/l |
| H⁺ en excès : | 0,9 à 1,1 mmol/l |
| chlorure : | 176 à 210 mmol/l |
| glucose : | 100 à 500 mmol/l. |

3. Solution pour la dialyse péritonéale selon l'une des revendications précédentes,
**caractérisée en ce que**
les solutions individuelles sont mises à disposition dans une poche à double compartiment qui est composée d'une poche en matière plastique qui comporte un premier compartiment contenant la première solution individuelle et un second compartiment contenant la seconde solution individuelle adjacents l'un à l'autre, les compartiments étant séparés l'un de l'autre par une soudure, qui est dimensionnée de telle sorte qu'elle s'ouvre lors d'une pression sur l'un des compartiment remplis de liquide et que le contenu des deux compartiments se mélange.
